(19) Europäisches Patentamt
European Patent Office
Office européen des brevets

(11) **EP 2 263 520 A1**

(12) # EUROPEAN PATENT APPLICATION

(43) Date of publication:
**22.12.2010 Bulletin 2010/51**

(51) Int Cl.:
**A61B 1/07** (2006.01)   **A61B 5/107** (2006.01)
**G01B 11/02** (2006.01)   **A61B 1/05** (2006.01)
**A61B 5/00** (2006.01)   *A61B 1/04* (2006.01)
**A61B 1/045** (2006.01)

(21) Application number: **10166288.0**

(22) Date of filing: **17.06.2010**

(84) Designated Contracting States:
**AL AT BE BG CH CY CZ DE DK EE ES FI FR GB
GR HR HU IE IS IT LI LT LU LV MC MK MT NL NO
PL PT RO SE SI SK SM TR**
Designated Extension States:
**BA ME RS**

(30) Priority: **18.06.2009 JP 2009144976**

(71) Applicant: **Fujifilm Corporation
Minato-ku
Tokyo (JP)**

(72) Inventor: **Shimotsu, Shinichi
Kanagawa (JP)**

(74) Representative: **HOFFMANN EITLE
Patent- und Rechtsanwälte
Arabellastraße 4
81925 München (DE)**

(54) **Endoscope system, endoscope, and method for measuring distance and illumination angle**

(57)     An endoscope (10) has a first light guide (22c) for transmitting measurement light from a light source (12) and a second light guide (22b) for transmitting IR light from an IR light source (12), and a CCD (24a, 24b) for capturing a projected shape of the measurement light. An image processing circuit (34a, 34b) extracts the pro-
jected shape from an image from the CCD, and calculates the size of the extracted projected shape to calculate a distance between an end surface (25) of the endoscope and an object of interest and an illumination angle of the IR light on a surface of the object of interest. A CPU (30) controls the operation of the IR light source in accordance with the calculated distance and the illumination angle.

FIG. 2

EP 2 263 520 A1

**Description**

FIELD OF THE INVENTION

The present invention relates to an endoscope system, an endoscope, and a method for measuring a distance and an illumination angle.

BACKGROUND OF THE INVENTION

Medical examinations using endoscopes have been common in the medical field. In endoscopy, maneuvers using various medical instruments such as an electric scalpel are performed. To perform the maneuvers smoothly, there is a demand to obtain a distance between the endoscope and an object of interest.

To improve visibility of blood vessels in submucosa in endoscopy, a contrast medium such as ICG (indocyanine green) is injected to an object of interest before the observation and then IR light is emitted to the object of interest. In a case where the IR light is used as the illumination, illumination intensity of the IR light, namely, the light intensity of the IR light incident on the object of interest, influences image quality of an in vivo image. According to the law of light attenuation, the illumination intensity is inversely proportional to the square of a distance between the object of interest and a light source. Therefore, it is particularly important to obtain distance information to control the illumination intensity in the observation using the IR light.

It is unsafe if the distance between the object of interest and the IR light source is too short, which makes the illumination intensity too high. In this regard, the distance information is also indispensable. An image obtained with the illumination of the IR light is the image of tissue below a surface of the object of interest, and the surface of the object of interest itself cannot be seen with the IR light. Without the distance information, the surface of the object of interest may be adversely affected with the IR light.

Conventionally, a method disclosed in Japanese Patent Laid-Open Publication No. 08-285541 is suggested as a method for measuring the distance between an object of interest and a light source. In Japanese Patent Laid-Open Publication No. 08-285541, a light exit end of distance measuring laser is disposed off-center from the center or optical axis of the illumination optical fiber. Laser beams parallel to the optical axis of the illumination light are emitted to the object of interest.

The illumination light is irradiated to the object of interest at a certain divergence angle relative to its optical axis. On the other hand, the distance measuring laser beams follow paths that are always a constant distance from the optical axis of the illumination light. As the distance between the object of interest and the light emission point increases, an imaging field of the distance measuring laser enlarges, and a projected position of the laser becomes relatively closer to the center of the image.

The distance between the laser exit and the object of interest is measured by the displacement of the projected position of laser from the center of the image.

In endoscopy, it is rare that the optical axis of the illumination light is orthogonal to a surface of an object of interest. Mostly, the illumination light is incident on the surface obliquely. Even at the same distances from the laser exits, the orthogonal incidence and the oblique incidence differ in the illumination intensity of the illumination light per unit area of the light incident surface on the object of interest. Particularly, the IR light observation requires precise control of the illumination intensity, and therefore necessitates the illumination angle information in addition to distance information.

In the method disclosed in Japanese Patent Laid-Open Publication No. 08-285541, information on illumination angles cannot be obtained, and the oblique incidence is not considered. Accordingly, the distance cannot be measured precisely in the case of the oblique incidence.

Adding a mechanism for obtaining information on the distance and the illumination angle to the endoscope increases a diameter of an insert section of the endoscope. As a result, a burden on a patient is increased. A diameter of a commercially available ultrasonic distance measuring sensor is, for example, approximately 10 mm. Adding such a sensor to the insert section substantially increases the diameter of the insert section, and therefore such sensor cannot be used for obtaining distance information.

SUMMARY OF THE INVENTION

An object of the present invention is to measure a distance between an exit end of illumination light and an object of interest and an illumination angle of the illumination light without an increase in the diameter of the insert section of the endoscope.

In order to achieve the above and other objects, an endoscope system of the present invention includes an endoscope and a calculating section. The endoscope illuminates an object of interest with illumination light from an end surface of the endoscope. The endoscope has a light guide and an image capturing section. The light guide transmits laser light and irradiates the object of interest with the laser light from the end surface. The image capturing section captures a projected shape of the laser light on the object of interest. The calculating section detects a size of the projected shape by analyzing the captured projected shape, and calculates a distance between the end surface and the object of interest and an illumination angle of the illumination light on the object of interest. The calculation of the distance and the illumination angle are performed based on the detected size.

It is preferable that the illumination light is infrared light.

It is preferable that the endoscope system further includes a control section for controlling an output of the illumination light in accordance with the calculated distance and the calculated illumination angle.

It is preferable that the control section controls the output such that illumination intensity on the object of interest is constant regardless of the distance and the illumination angle.

It is preferable that the control section turns off the illumination light when the calculated distance is equal to or less than a threshold value.

It is preferable that the light guide has a refractive index profile for substantially collimating the laser light, and a divergence angle of the substantially collimated light is smaller than that of the illumination light.

It is preferable that in a case where a surface of the object of interest is orthogonal to an optical axis of the laser light, an outer shape of the projected shape formed on the surface of the object of interest is substantially circular, and the size refers to a radius of the substantially circular projected shape.

It is preferable that in a case where a surface of the object of interest is tilted relative to the optical axis of the laser light, the outer shape of the projected shape formed on the surface of the object of interest is substantially elliptical, and the size refers to a minor radius and a major radius of the substantially elliptical projected shape.

It is preferable that the light guide is disposed such that the projected shape is formed outside of an area illuminated with the illumination light.

It is preferable that the endoscope system further includes a display control section for displaying the calculated distance and the calculated illumination angle together with the image of the object of interest illuminated with the illumination light on a monitor.

It is preferable that the endoscope further includes a second light guide for transmitting the illumination light to the end surface, and the second light guide has a tapered section tapered toward the end surface.

An endoscope of the present invention includes a light guide and an image capturing section. The light guide transmits laser light and irradiates the object of interest with the laser light from the end surface. The image capturing section captures a projected shape of the laser light on the object of interest. A distance between the end surface and the object of interest and an illumination angle of the illumination light from the end surface on the object of interest are calculated based on a size of the projected shape. The size is detected by analyzing the captured projected shape.

A method for measuring a distance between an end surface of an endoscope and an object of interest and an illumination angle of illumination light from the end surface on the object of interest includes a transmitting step, a capturing step, a detecting step, and a calculating step. In the transmitting step, laser light is transmitted through a light guide and the object of interest is irradiated with the laser light from the end surface. In the capturing step, a projected shape of the laser light on the object of interest is captured. In the detecting step, a size of the projected shape is detected by analyzing the captured projected shape. In the calculating step, the distance and the illumination angle are calculated based on the detected size.

According to the present invention, a distance between an end surface of an endoscope and an object of interest and an illumination angle of illumination light from the end surface on the object of interest are calculated based on an image obtained by capturing a projected shape of laser light. As a result, the distance and the illumination angle are measured without the increase in the diameter of the insert section of the endoscope.

BRIEF DESCRIPTION OF THE DRAWINGS

The above and other objects and advantages of the present invention will be more apparent from the following detailed description of the preferred embodiments when read in connection with the accompanied drawings, wherein:

Figure 1 is a schematic view of an endoscope system;
Figure 2 is a block diagram showing an internal configuration of the endoscope system;
Figure 3 is a partially enlarged cross-sectional view showing a distal portion of an insert section of an endoscope;
Figure 4 is an explanatory view of a projected shape of measurement light on a surface, of an object of interest, parallel to an end surface of the insert section;
Figure 5 is an explanatory view of a projected shape of the measurement light on the surface, of the object of interest, tilted relative the end surface of the insert section;
Figure 6 is a block diagram showing an internal configuration of an image processing circuit;
Figure 7 is a graph showing an example of controlling an IR light output;
Figure 8 is a flowchart showing processing steps of the endoscope system;
Figures 9A and 9B are partially enlarged cross-sectional views of distal portions of insert sections of endoscopes in other embodiments; and
Figure 10 shows an example of displaying a distance and an illumination angle.

DESCRIPTION OF THE PREFERRED EMBODIMENTS

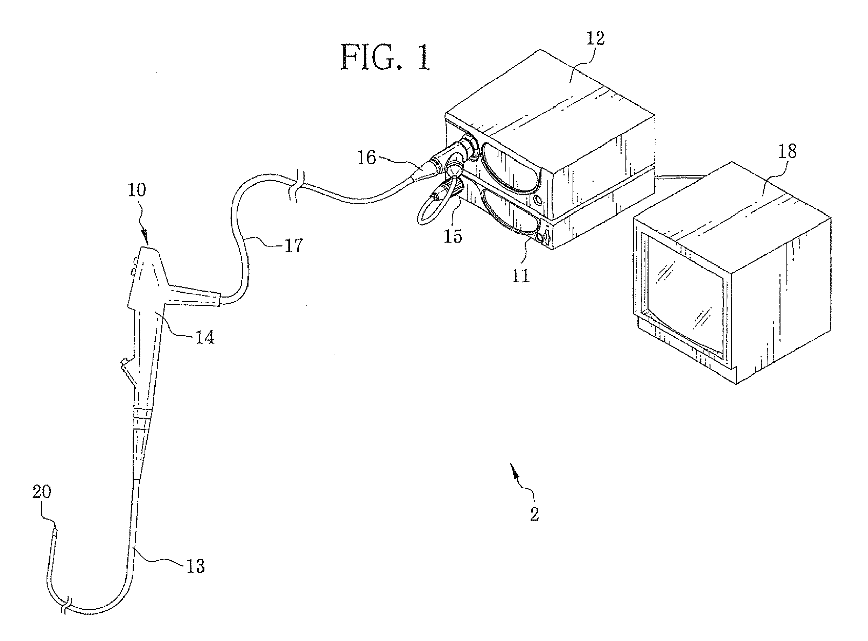

In Fig. 1, an endoscope system 2 is composed of an endoscope 10, a processor apparatus 11, and a light source apparatus 12. The endoscope 10 has a flexible insert section 13 to be inserted into a body of a patient, a handling section 14, a processor connector 15, a light source connector 16, and a universal cord 17. The handling section 14 is provided in a base portion of the insert section 13. The processor connector 15 is connected to the processor apparatus 11. The light source connector 16 is connected to the light source apparatus 12. The handling section 14, the processor connector 15, and the light source connector 16 are connected via the universal cord 17.

The processor apparatus 11 is electrically connected to the light source apparatus 12, and controls overall operations of the endoscope system 2. The processor apparatus 11 supplies power to the endoscope 10 via cables inserted through the universal cord 17 and the insert section 13. The endoscope 10 obtains image signals of an image of an object of interest. The processor apparatus 11 performs various image processing to the obtained image signals and generates an image. A monitor 18 is connected to the processor apparatus 11 via a cable. The image generated in the processor apparatus 11 is displayed on the monitor 18 as an in vivo image.

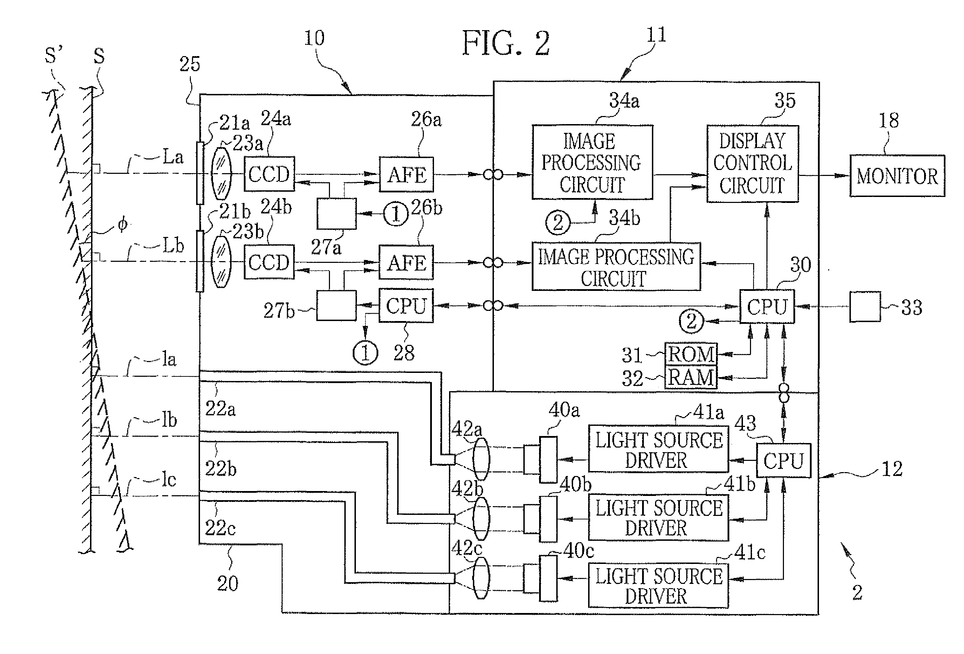

In Fig. 2, two image capturing windows 21a and 21b, and three exit ends of light guides 22a, 22b and 22c are disposed on an end surface 25 of a distal portion 20 of the insert section 13. CCDs 24a and 24b for in vivo imaging are situated behind the image capturing windows 21a and 21b via objective optical systems 23a and 23b, respectively. Each of the objective optical systems 23a and 23b is composed of a lens group and a prism. Hereinafter, in drawings and descriptions, a part or member for use with normal light (white light) has a numeral with a suffix "a". A part or member for use with infrared light (hereinafter referred to as IR light) has a numeral with a suffix "b". A part or member for use with measurement light has a numeral with a suffix "c". The measurement light is used for measuring a distance between the end surface 25 of the distal portion 20 and the object of interest and an illumination angle of illumination light (the normal light or the IR light) illuminated on the object of interest from the end surface 25.

The CCDs 24a and 24b are of the interline transfer type, for example. The CCDs 24a and 24b are CCD image sensors using progressive scanning. An in vivo image of the object of interest is formed on an imaging surface of the CCD 24a via the image capturing window 21a and the objective optical system 23a, and/or on an imaging surface of the CCD 24b via the image capturing window 21b and the objective optical system 23b. The CCD 24a has ISO sensitivity in the visible range. The CCD 24b has ISO sensitivity in the infrared range.

The light guide 22a transmits the normal light from the light source apparatus 12. The light guide 22b transmits the IR light from the light source apparatus 12. The light guide 22c transmits the measurement light from the light source apparatus 12. The light guides 22a to 22c illuminate the object of interest with the transmitted light.

The objective optical systems 23a and 23b and the light guides 22a to 22c are disposed such that optical axes La and Lb of the objective optical systems 23a and 23b, and optical axes 1a, 1b and 1c of the light from the light guides 22a to 22c are parallel to each other, and that all the optical axes La, Lb, 1a, 1b, and 1c are orthogonal to the end surface 25 of the distal portion 20. In a case where a surface S of the object of interest is parallel to the end surface 25 of the distal portion 20, each of the optical axes La, Lb, 1a, 1b, and 1c is orthogonal (incident angle $\phi = 0°$) to the surface S of the object of interest. In a case where a surface S' of the object of interest is tilted relative to the end surface 25 of the distal portion 20, each of the optical axes La, Lb, 1a, 1b, and 1c is also tilted relative to the surface S' of the object of interest.

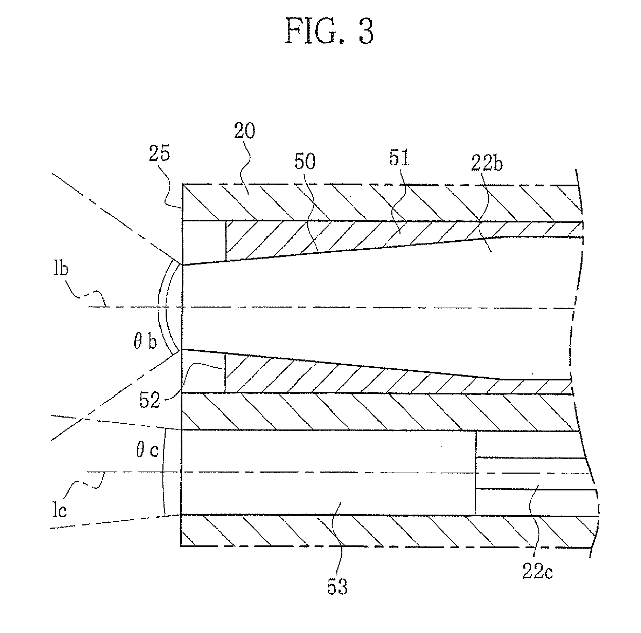

In Fig. 3, the light guide 22b has a tapered section 50 formed in the vicinity of an exit end of the light guide 22b, namely, in the vicinity of the end surface 25. In the tapered section 50, a diameter of the light guide 22b is gradually tapered toward the end surface 25. The tapered section 50 is formed by heating and stretching a distal end or tip of an optical fiber of the light guide 22b. The tapered section 50 has a constant tapered angle with respect to the optical axis 1b. The optical axis 1b coincides with centers of cross-sectional circles of the tapered section 50.

A core diameter and a clad diameter of the optical fiber of the light guide 22b other than the tapered section 50 are, for example, 230 $\mu$m and 250 $\mu$m, respectively. A core diameter and a clad diameter of the optical fiber at the exit end, namely, on the end surface 25 of the distal portion 20 of the light guide 22b are, for example, 46 $\mu$m and 50 $\mu$m, respectively. Here, a taper rate (a diameter ratio of a core diameter of the optical fiber at the exit end of the tapered section 50/ a core diameter of the optical fiber other than the tapered section 50) is 0.2.

The light guide 22b has optical transmission properties. The light guide 22b is adhered inside the distal portion 20 with a polymer adhesive agent 51 which is hardened or cured with UV rays. The adhesive agent 51 is filled between the distal portion 20 and the light guide 22b except for the end surface 25 side of the tapered section 50. An outer circumferential surface of the light guide 22b to which the adhesive agent 51 is not applied is exposed to air. A substantially ring-like hollow 52 is formed between the distal portion 20 and the exposed outer circumferential surface of the light guide 22b in the vicinity of the end surface 25.

The adhesive agent 51 has a lower refractive index than a clad of the optical fiber of the light guide 22b. For example, the refractive index of the clad is not less than 1.43 and not more than 1.44, and the refractive index of the adhesive agent 51 is not less than 1.40 and not more than 1.41.

The IR light transmits through the core by total internal reflection at the interface between the core and the clad of an optical fiber of the light guide 22b. On the other hand, in the tapered section 50, the light incident angle of the IR light onto the clad becomes smaller than that in a section other than the tapered section 50. As a result, a part of the IR light is not totally reflected at the interface between the core and the clad, and leaks to the clad. The IR light leaked to the clad is totally reflected at an interface between the clad and the adhesive agent 51 by total internal reflection, and is again transmitted through the tapered section 50.

The IR light transmitted through the tapered section 50 is output from the end surface 25. A part of the IR light is output from an exposed exit portion of the light guide 22b in the hollow 52. In the hollow 52, the exposed portion directly contacts with air. A critical angle for the total internal reflection at the interface between the clad and air is larger than a critical angle at the interface between the clad and the adhesive agent 51. As a result, the divergence angle θb and a numerical aperture (NA) of the IR light become larger than in the case where the tapered section 50 and the hollow 52 are not provided. The divergence angle or FWHM (abbreviation for Full Width Half Maximum) θb is an angle of the output light at which the illumination intensity at a far field pattern (an image of the IR light formed on a surface approximately 10 cm away from and parallel to the end surface 25) is half the maximum value. Thereby, the area illuminated with the IR light becomes larger than the view field of the CCD 24b. It should be noted that the divergence angle has the horizontal component and the vertical component. The horizontal component and the vertical component of the divergence angle of the IR light are substantially identical values (angles). Accordingly, the projected shape of the IR light on the surface S, of the object of interest, parallel to the end surface 25 of the distal portion 20 is substantially circular in shape. This is the same with the normal light and the measurement light.

The light guide 22c is disposed directly below the light guide 22b. The light guide 22c is, for example, an optical fiber of 125 μm in diameter. An exit end of the light guide 22c is connected to a graded index (GI) section 53 or refractive index profile device. The GI section 53 is a graded index (GI) fiber or a GRIN lens having a refractive index profile in a diameter direction, namely, the refractive index gradually decreases outward from the optical axis center. The measurement light transmitted through the light guide 22c is substantially collimated or made substantially parallel in the GI section 53, and then output from the end surface 25 with a sufficiently small divergence angle θc (for example, in a range from approximately 3° to 6°) relative to the divergence angle θb of the IR light.

In Fig. 2, the endoscope 10 is provided with analog front ends (hereinafter abbreviated as AFEs) 26a and 26b, CCD drivers 27a and 27b, and a CPU 28. Each of the AFEs 26a and 26b is composed of a correlated double sampling circuit (hereinafter abbreviated as CDS), an automatic gain control circuit (hereinafter abbreviated as AGC), and an analog/digital converter (hereinafter abbreviated as A/D) (all not shown). The CDSs perform correlated double sampling processing to image signals output from the CCDs 24a and 24b, respectively. Thereby, reset noise and amplification noise generated in the CCDs 24a and 24b are removed from the image signals. Then, the AGCs amplify the image signals with a gain (amplification factor) designated by the processor apparatus 11. Thereafter, the A/Ds convert the image signals into digital image signals with predetermined bits. The digital image signals are input to the processor apparatus 11 via the universal cord 17 and the processor connector 15.

The CCD driver 27a generates drive pulses for the CCD 24a, and synchronous pulses for the AFE 26a. The CCD driver 27b generates drive pulses for the CCD 24b, and synchronous pulses for the AFE 26b. The drive pulses include vertical and horizontal scan pulses, an electronic shutter pulse, a read pulse, and a reset pulse. The CCD 24a and 24b perform image capture operations in response to the drive pulses from the CCD drivers 27a and 27b, respectively, and output the image signals. The AFEs 26a and 26b operate based on the synchronous pulses from the CCD drivers 27a and 27b, respectively.

After the endoscope 10 and the processor apparatus 11 are connected, the CPU 28 actuates the CCD drivers 27a and 27b, and adjusts the gains of the AGCs in the AFEs 26a and 26b based on the operation start instruction from a CPU 30 of the processor apparatus 11.

The CPU 30 controls the overall operations of the processor apparatus 11. The CPU 30 is connected to each section via a data bus, an address bus, and control lines (all not shown). Various programs such as OS and application programs for controlling the operation of the processor apparatus 11 and data such as graphic data are stored in a ROM 31. The CPU 30 reads necessary programs and data from the ROM 31, and expands them in a RAM 32 that is a working memory, and executes the read program sequentially. The CPU 30 obtains information which varies in every examination, for example, text information such as examination dates and information on patients and operators, through a front panel 33 of the processor apparatus 11 or a network such as LAN (Local Area Network). The obtained information is stored in the RAM 32.

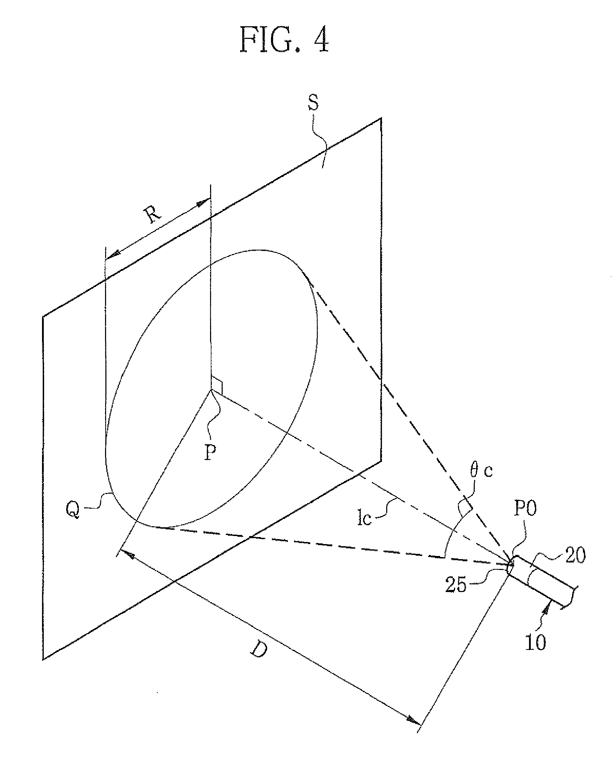

Each of image processing circuits 34a and 34b performs image processing, such as color interpolation, white balance adjustment, gamma correction, image enhancement, image noise reduction, and color conversion to the input digital image signal to generate an image. In addition, the image processing circuit 34a calculates a distance D (see Fig. 4) between the end surface 25 of the distal portion 20 and an object of interest, and an illumination angle φ of the IR light based on analysis results of the measurement light.

A display control circuit 35 receives from the CPU 30 the graphic data stored in the ROM 31 and the RAM 32. The graphic data includes a display mask which covers an ineffective pixel area and exposes an effective pixel area of an in vivo image, the text information such as the examination dates and information on patients and operators, and GUI (Graphical User Interface). The display control circuit 35 performs various display control processing, such as image superimposition of the display mask, the text information, and the GUI onto the images from the image processing circuits 34a and 34b, and graphic drawing processing to a display screen on the monitor 18.

The display control circuit 35 has a frame memory which temporarily stores the images from the image processing circuit 34a and 34b. The display control circuit 35 reads an image from the frame memory and converts the read image into video signals, such as component signals or composite signals, corresponding to a display format of the monitor 18. Thereby, an in vivo image is displayed on the monitor 18.

Additionally, the processor apparatus 11 is provided with a data compression circuit, a media I/F, a network I/F (not shown), and the like, and they are connected to the CPU 30 via the data bus and the like. The data compression circuit performs image compression in a predetermined compression format, for example, JPEG format. The media I/F stores the compressed image in a removable medium such as a CF card, a magneto optical disk (MO), or a CD-R. The network I/F controls various data transmission between the processor apparatus 11 and a network such as a LAN.

The light source apparatus 12 has light sources 40a, 40b, and 40c. The light source 40a is a xenon lamp or a white LED (light emitting diode) which generates normal light with different wavelengths from red to blue, for example, light in a broad wavelength range of not less than 480 nm and not more than 750 nm. The light source 40b is a semiconductor laser or the like having an output of not less than 100 mW and generates IR light. The light source 40b generates, for example, so-called near IR light, that is, light in a narrow-band in a wavelength range of not less than 750 nm and not more than 2500 nm. The light source 40c is a semiconductor laser or the like, and generates blue light having light intensity only at around 445 nm, for example. In this case, the projected shape of the measurement light is captured with the CCD 24a.

The light sources 40a to 40c are driven by light source drivers 41a, 41b, and 41c, respectively. A condenser lens 42a collects light from the light source 40a, and transmits the collected light to a light incident end of the light guide 22a. A condenser lens 42b collects light from the light source 40b, and transmits the collected light to a light incident end of the light guide 22b. A condenser lens 42c collects light from the light source 40c, and transmits the collected light to a light incident end of the light guide 22c. A CPU 43 communicates with the CPU 30 of the processor apparatus 11, and controls operations of the light source drivers 41a to 41c.

The endoscope system 2 has a normal mode and an infrared mode (hereinafter referred to as IR mode). In the normal mode, an image is captured with the normal light. In the IR mode, an image is captured with the IR light. In the IR mode, a contrast medium such as ICG (indocyanine green) is injected into an object of interest, and then blood vessels in the submucosa of the object of interest are observed. The normal mode or the IR mode is selected by, for example, operating the front panel 33 of the processor apparatus 11.

In a case where the normal mode is selected, the CPU 30 actuates the CCD 24a, the AFE 26a, image processing circuit 34a, and the like. The CPU 30 controls the light source drivers 41a to 41c via the CPU 43 to turn on the light source 40a and turn off the light sources 40b and 40c. Thereby, the obj ect of interest is illuminated only with the normal light. On the monitor 18, an image captured with the illumination of the normal light is displayed alone as an in vivo image.

On the other hand, in a case where the IR mode is selected, the CPU 30 actuates the CCD 24b, the AFE 26b, and the image processing circuit 34b, and turns on all the light sources 40a to 40c. The object of interest is illuminated with the normal light, the IR light, and the measurement light. On the monitor 18, the display control circuit 35 displays two images, one captured with the illumination of the normal light and the other captured with the illumination of the IR light, separately.

In Fig. 4, in a case where the surface S of the object of interest is parallel to the end surface 25 of the distal portion 20, a projected shape Q of the measurement light on the surface S is substantially circular in shape. "P0" is a light exit point of the measurement light. A point "P" is an intersection point of the surface S and the normal from the light exit point P0 to the surface S, namely, the intersection point of the surface S and the optical axis 1c. The point P is also the center of the circle of the projected shape Q. "R" is a radius of the projected shape Q. In a case where the distance D between the light exit point P0 and the point P is 100 mm, the radius R is in a range from 2.5 mm to 5 mm, for example. The measurement light is emitted in a conical form. This conical form has the projected shape Q as the base, the point P0 as the apex or vertex, the distance D as the height.

The size of the projected shape Q increases as the distance D between the end surface 25 (light exit point P0) and the object of interest (point P) increases. Conversely, the size of the projected shape Q decreases as the distance D decreases. The distance D is obtained by a mathematical expression (1) using a divergence angle $\theta c$ of the measurement light and the radius R of the projected shape Q.

$$(1) \quad D = R / \tan (\theta c / 2)$$

The divergence angle $\theta c$ of the measurement light is easily obtained by actually measuring the distance D and the radius R of the projected shape Q, and substituting the measured values into the mathematical expression (1). Alternatively, the divergence angle $\theta c$ is obtained based on the specifications of the light source 40c, the light guide 22c, and the GI section 53. Thus, the divergence angle $\theta c$ becomes a known quantity. Accordingly, the distance D between the end surface 25 (the light exit point P0) and the object of interest (the point P) is calculated only with the radius R of the projected shape Q.

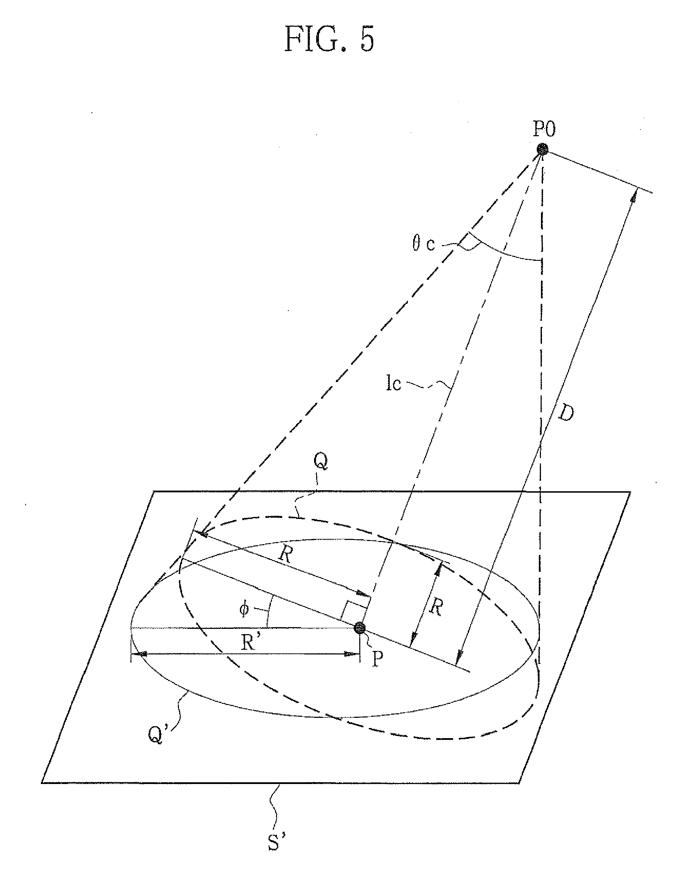

In Fig. 5, the projected shape Q' of the measurement light is formed in a substantially elliptical shape on the surface S', of the object of interest, tilted relative to the end surface 25. The projected shape Q' is a slanted cross section of cone-shaped beams of the measurement light. A center of the projected shape "Q'" is the point P as with the projected shape Q. A minor radius of the projected shape Q' is the same as the radius R of the projected shape Q. A major radius of the projected shape Q' is R'. To be more precise, the center of the projected shape Q' is slightly shifted from the point P. However, since the divergence angle θc is extremely small, the difference between the center of the projected shape Q' and the point P can be ignored. The center of the projected shape Q' is approximated to the point P. An illumination angle φ of the measurement light is calculated using a mathematical expression (2).

$$(2) \quad \phi = \cos^{-1} (R/R')$$

With the use of the mathematical expression (2), the calculation of the illumination angle φ of the measurement light only requires values of the major radius R' and the minor radius R of the elliptical projected shape Q'. The distance D is calculated using the mathematical expression (1) as in the case of the surface S. The projected shapes Q and Q' may be deformed if the surfaces S and S' are uneven. However, the projected shapes Q and Q' are extremely small in size, for example, a radius or a minor radius may be in a range from 2.5 mm to 5 mm, so the distortions caused by the uneven surfaces may be ignored.

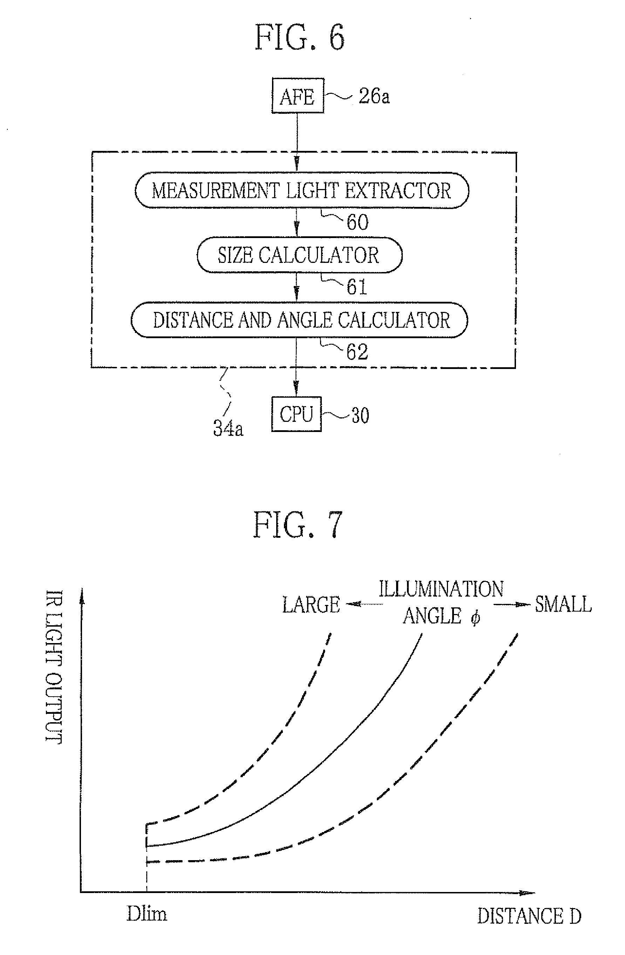

In Fig. 6, in a case where the IR mode is selected, a measurement light extractor 60, a size calculator 61, and a distance and angle calculator 62 are created in the image processing circuit 34a.

Using a known image recognition technology, the measurement light extractor 60 extracts a projected shape of the measurement light from an image illuminated with the normal light and containing the projected shape of the measurement light. This image is input from the AFE 26a. The measurement light extractor extracts blue pixel components, namely, the pixel components of the measurement light, from the input image. Of the extracted blue pixel components, those having larger pixel values than the other extracted blue pixel components and concentrated in a substantially circular or substantially elliptical area are extracted as the projected shape of the measurement light. It is relatively easy to extract an area or a surface from which the blue measurement light is reflected, because most of the organs in the body cavity are red.

The size calculator 61 calculates the size (here, the size refers to the minor radius R and the major radius R' , or the radius R) of the projected shape of the measurement light extracted by the measurement light extractor 60. A unit of the size of the projected shape of the measurement light is the number of pixels. The distance and angle calculator 62 substitutes the values, obtained by the size calculator 61, into the mathematical expressions (1) and (2). Thus, the illumination angle φ and the distance D based on the number of pixels are calculated. As described above, the divergence angle θc of the measurement light is a known quantity. Only the radius or the minor radius R is substituted into the mathematical expression (1). Alternatively or in addition, relations between the size (the number of pixels) and the actual dimensions of the projected shape may be obtained in advance, and an actual distance D may be calculated based on the obtained relations.

According to the law of light attenuation, the illumination intensity of the IR light on the surface of the object of interest abruptly decreases as the distance D increases. In addition, the IR illumination intensity per unit area on the surface of the object of interest decreases as the illumination angle φ increases. When the distance D is below a predetermined threshold value, the object of interest is adversely affected by the IR light. The CPU 30 controls the drive of the light source 40b via the CPU 43 in view of the above IR light properties and the calculation results of the distance and angle calculator 62.

As shown in Fig. 7 as an example, the CPU 30 rapidly increases an output (IR light output) of the light source 40b as the distance D increases so as to compensate for the attenuation of illumination intensity of the IR light and make the illumination intensity constant on the surface of the object of interest regardless of the distance D. The CPU 30 increases an amount of change in the IR light output relative to the distance D as the illumination angle φ increases. The CPU 30 turns off the light source 40b when the distance D is equal to or smaller than a threshold value Dlim.

The information for controlling the IR light output, shown in Fig. 7 as an example, is previously stored as a data table or a function in the ROM 31. The CPU 30 reads this control information from the ROM 31, and sends the CPU 43 a control signal based on this control information and the calculation results of the distance and angle calculator 62. The CPU 43 controls the drive of the light source 40b via the light source driver 41b based on the control signal from the CPU 30.

Next, an operation of the above-configured endoscope system 2 is described. To observe a body cavity of a patient using the endoscope 10, an operator connects the endoscope 10 to the processor apparatus 11 and the light source apparatus 12. The operator turns on the endoscope 10, the processor apparatus 11 and the light source apparatus 12. Through the front panel 33 and the like, the operator inputs patient information and instructs the start of the endoscopy.

The operator inserts the insert section 13 in the body cavity of the patient. On the monitor 18, the operator observes an in vivo image captured with the CCD 24a or the CCD 24b while the body cavity is illuminated with the normal light or the IR light from the light source apparatus 12.

The image signals output from the CCD 24a are subjected to various processing in the AFE 26a, and then input to the image processing circuit 34a of the processor apparatus 11. In the image processing circuit 34a, the image signals are subjected to various image processing to generate an image. The generated image is input to the display control circuit 35. The image signals output from the CCD 24b are subjected to various processing in the AFE 26b, and then input to the image processing circuit 34b of the processor apparatus 11. In the image processing circuit 34b, the image signals are subjected to various image processing to generate an image. The generated image is input to the display control circuit 35. In the display control circuit 35, the image is subjected to various display control processing based on the graphic data from the CPU 30. Thus, the in vivo image is displayed on the monitor 18.

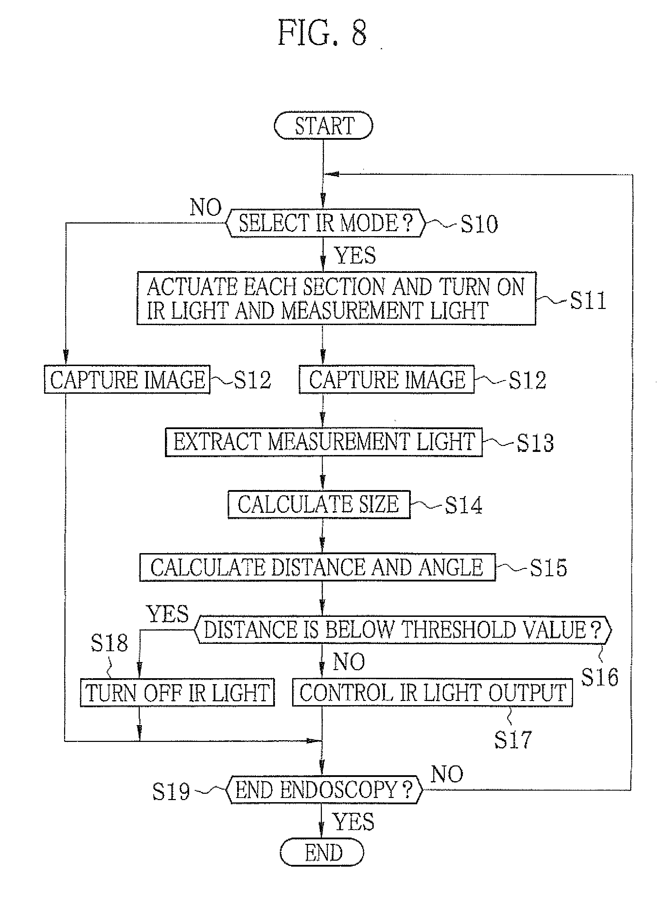

In Fig. 8, in a case where the IR mode is selected (YES in S10), the CCD 24b, the AFE 26b, and the image processing circuit 34b, and the like are actuated, and all the light sources 40a to 40c are turned on (S11). In the image processing circuit 34a, the measurement light extractor 60, the size calculator 61, and the distance and angle calculator 62 are created.

An image (normal image) illuminated with the normal light and an image (IR image) illuminated with the IR light are captured with the CCDs 24a and 24b, respectively (S12). The captured normal image passes through the AFE 26a and the image processing circuit 34a. The captured IR image passes through the AFE 26b and the image processing circuit 34b. Then, the normal image and the IR image are displayed on the monitor 18 separately by the display control circuit 35. The normal image captured with the CCD 24a contains the projected shape of the measurement light.

In the image processing circuit 34a, the measurement light extractor 60 extracts the projected shape of the measurement light from the image (digital image signals) input from the AFE 26a (S13). Then, the size calculator 61 calculates the size of the extracted projected shape (S14). The distance and angle calculator 62 calculates the distance D between the end surface 25 and the object of interest and the illumination angle φ (S15).

The CPU 30 receives the calculation results of the distance and angle calculator 62. In accordance with the calculation results, the CPU 30 controls the IR output via the CPU 43 such that the intensity of the IR light at the object of interest becomes constant regardless of the distance D and the illumination angle φ (S17). When the distance D is below the threshold value Dlim (YES in S16), the CPU 30 turns off the light source 40b (S18).

These steps are repeated until the end of the endoscopy is instructed (YES in S19) or the normal mode is selected (NO in S10).

In a case where the normal mode is selected (NO in S10), the CCD 24b, the AFE 26b, and the image processing circuit 34b are not actuated. Only the light source 40a is turned on. The obj ect of interest is illuminated only with the normal light. The image captured only with the normal light is displayed on the monitor 18.

As described above, the object of interest is illuminated with the measurement light from the light source 40c via the light guide 22c, and the projected shape of the measurement light is captured with the CCD 24a. The size of the projected shape is calculated or detected by analyzing the obtained image. Based on the calculation results or detected results, the illumination angle φ and the distance D between the object of interest and the end surface 25 of the endoscope 10 are obtained. Thus, information on the distance D and the illumination φ is obtained without increasing the diameter of the insert section 13.

The IR light output is controlled in accordance with the calculated distance D and the illumination angle φ such that the illumination intensity of the IR light on the surface of the object of interest is constant throughout the IR mode. Turning off the light source 40b when the distance D is below the threshold value Dlim ensures safety in the endoscopy without adversely affecting the object of interest by the IR light.

In the above embodiment, the light guides 22b and 22c are disposed such that the optical axes 1b and 1c become parallel to each other. Alternatively, the optical axes 1b and 1c may not necessarily be parallel to each other. The optical axis 1c may be tilted within a range in which the projected shape is inside a field view of the CCD 24a and the measurement light impinges on an area, of the object of interest, outside the area illuminated with the IR light.

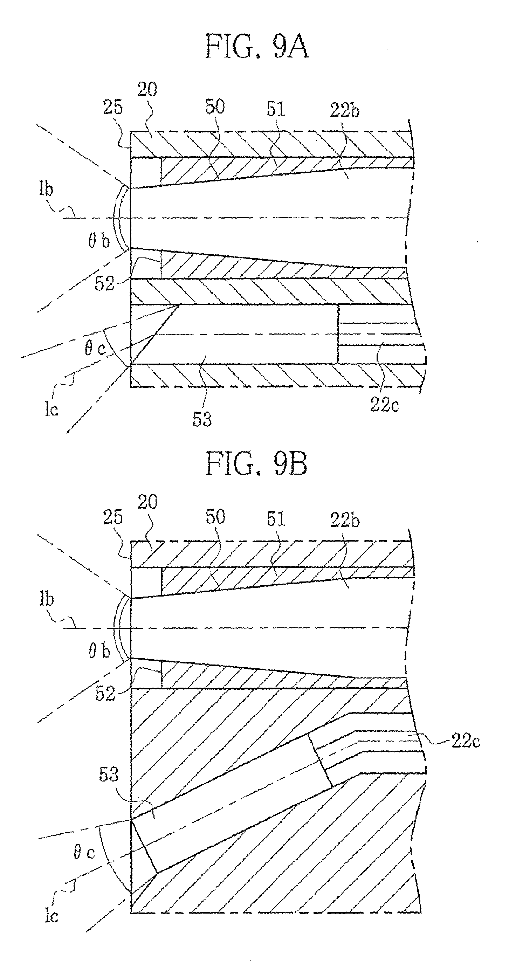

As shown in Fig. 9A, to tilt the optical axis 1c outward, a distal end of the GI fiber or the GRIN lens of the GI section 53 is cut obliquely relative to the end surface 25 of the endoscope 10. Alternatively or in addition, as shown in Fig. 9B, the light guide 22c and the GI section 53 may be disposed obliquely relative to the end surface 25. In any case, it is necessary to adjust the illumination angle φ, calculated using the mathematical expression (2), in accordance with the tilt angle of the optical axis 1c relative to the optical axis 1b.

The projected shape of the measurement light can be extracted without influence of the IR light when the measurement light impinges on an area, of the surface of the object of interest, outside the area illuminated with the IR light. Thereby, the distance D and the illumination angle φ are measured with higher accuracy.

Alternatively, both the illumination light and the measurement light may be in the same wavelength range, for example, an IR wavelength range. In a case where the illumination light and the measurement light is the IR light, the number of parts used in the light source 40c may be reduced by splitting a part of the light guide 22b and using the split light guide as the light guide 22c. In this case, the projected shape of the measurement light is captured with the CCD 24b. The measurement light extractor 60, the size calculator 61, and the distance and angle calculator 62 shown in Fig. 6 are created in the image processing circuit 34b. Penetration depth of light into the object of interest differs according to the wavelength of the light. In a case where IR light is used as the measurement light, the distance D is corrected in consideration of a difference in the penetration depth from the above described blue light with the wavelength of 445 nm.

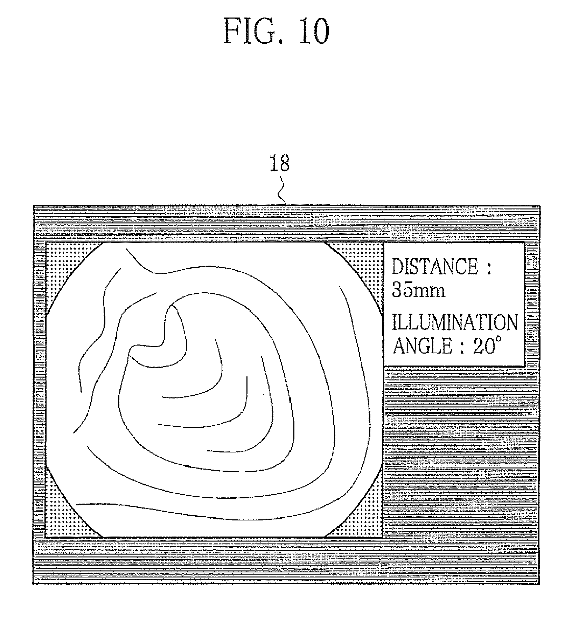

In the above embodiment, the endoscope using the normal light and the IR light as the illumination light is described as an example. The present invention is also applicable to the common endoscope 10 using only the normal light as the illumination light. In this case, unlike the above embodiment, the output of the illumination light is not necessarily controlled precisely. As shown in Fig. 10, the display control circuit 35 may display the distance D (actual distance) and the illumination angle φ on the monitor 18 together with the in vivo image. The displayed distance D and the illumination angle φ are useful in maneuvering medical instruments. In a case where a rigid scope is used, the distance D and the illumination angle φ indicate a correct observation position. The distance D and the illumination angle φ may be displayed on the monitor 18 in the above embodiment where the normal light and the IR light is used as the illumination light.

In the above embodiment, the distance D and the illumination angle φ are calculated to control the IR light output. However, the relations between the radius or the minor radius R, the major radius R', the distance D, and the illumination angle φ are obtained from the mathematical expressions (1) and (2). Therefore, obtaining the values of the radius or the minor radius R and the major radius R' is substantially the same as the calculation of the distance D and the illumination angle φ, namely, the IR light output can be controlled based on the values of the radius or the minor radius R and the major radius R'. In this case, it is not necessary to calculate the distance D and the illumination angle φ.

The projected shape of the measurement light is not limited to a circular shape. The projected shape may be in the shape of a cross, or a ring having the outer perimeter of the illumination light as an inner edge of the ring. The measurement light may be spotlight emitted onto each apex of a regular triangle as in 3-spot lighting. The projected shape may take any shape as long as 2-dimensional measurement(s), e.g. the radius or the minor radius R and the major radius R', of the projected shape can be obtained. To form a ring-shaped projected shape, laser beams are incident obliquely on a cylinder having a mirror-like inner wall and transmitted helically while being reflected off the inner wall.

In the above embodiment, the light source 40b is turned off when the distance D is below the threshold value Dlim. Alternatively, the IR output may be lowered to a level which does not adversely affect the object of interest, or a warning may be displayed on the monitor 18. Thereby, a minimum view field is ensured with the IR light even if the distance D is below Dlim.

A sampling rate for calculating the distance D and the illumination angle φ to control the IR light output is not particularly described. The calculation may be performed every frame, or at a certain time interval. The calculation may be performed upon the operation of an operator. A speed of motion of the distal portion 20 may be detected with an acceleration sensor or the like. The calculation may be performed every frame when the distal portion 20 moves fast, and at a certain time interval when the distal portion 20 moves slowly or stops. In a case where the calculation is performed at a certain time interval, the measurement light may blink in accordance with the time interval.

In the above embodiment, parts or members related to the measurement light such as the light guide 22c are integrally provided in the endoscope 10 or the light source apparatus 12. Alternatively, such members may be provided separately from the endoscope 10 or the light source apparatus 12. In this case, the light guide 22c is covered with a sheath, and this sheath is inserted through a forceps channel to place the exit end of the light guide 22c at the end surface 25. The light guide 22c may be attached to the distal portion 20 and the end surface 25 using a specific holder or the like.

Various changes and modifications are possible in the present invention and may be understood to be within the present invention.

## Claims

1. An endoscope system (2) comprising:

an endoscope (10) which illuminates an object of interest with illumination light from an end surface (25) of the endoscope, the endoscope including:

a light guide (22c) for transmitting laser light and irradiating the object of interest with the laser light from the end surface; an image capturing section (24a, 24b) for capturing a projected shape of the laser light on the object of interest; and

a calculating section (61, 62) for detecting a size of the projected shape by analyzing the captured projected shape, and calculating a distance between the end surface and the object of interest and an illumination angle of the illumination light on the object of interest, the calculation of the distance and the illumination angle being performed based on the detected size.

2. The endoscope system of claim 1 or 2, wherein the illumination light is infrared light.

3. The endoscope system of claim 1, further including a control section (30) for controlling an output of the illumination light in accordance with the calculated distance and the calculated illumination angle.

4. The endoscope system of claim 3, wherein the control section controls the output such that illumination intensity on the object of interest is constant regardless of the distance and the illumination angle.

5. The endoscope system of claim 3, wherein the control section turns off the illumination light when the calculated distance is equal to or less than a threshold value.

6. The endoscope system of any one of the preceding claims, wherein the light guide has a refractive index profile for substantially collimating the laser light, and a divergence angle of the substantially collimated light is smaller than that of the illumination light.

7. The endoscope system of any one of the preceding claims, wherein in a case where a surface of the object of interest is orthogonal to an optical axis of the laser light, an outer shape of the projected shape formed on the surface of the object of interest is substantially circular, and the size refers to a radius of the substantially circular projected shape.

8. The endoscope system of claim 7, wherein in a case where a surface of the object of interest is tilted relative to the optical axis of the laser light, the outer shape of the projected shape formed on the surface of the object of interest is substantially elliptical, and the size refers to a minor radius and a major radius of the substantially elliptical projected shape.

9. The endoscope system of any one of the preceding claims, wherein the light guide is disposed such that the projected shape is formed outside of an area illuminated with the illumination light.

10. The endoscope system of any one of the preceding claims, further comprising a display control section (35) for displaying the calculated distance and the calculated illumination angle together with the image of the object of interest illuminated with the illumination light on a monitor (18).

11. The endoscope system of any one of the preceding claims, wherein the endoscope further includes a second light guide (22b) for transmitting the illumination light to the end surface, and the second light guide has a tapered section tapered toward the end surface.

12. An endoscope (10) which illuminates an object of interest with illumination light from an end surface (25) of the endoscope, the endoscope comprising:

a light guide (22c) for transmitting laser light and for irradiating the object of interest with the laser light from the end surface; and an image capturing section (24a, 24b) for capturing a projected shape of the laser light on the object of interest, a distance between the end surface and the object of interest and an illumination angle of the illumination light from the end surface on the object of interest being calculated based on a size of the projected shape, the size being detected by analyzing the captured projected shape.

13. A method for measuring a distance between an end surface (25) of an endoscope (10) and an object of interest and an illumination angle of illumination light from the end surface on the object of interest, the method comprising the steps of:

transmitting laser light through a light guide (22c) and irradiating the object of interest with the laser light from the end surface; capturing a projected shape of the laser light on the object of interest; detecting a size of the projected shape by analyzing the captured projected shape; and calculating the distance and the illumination angle based on the detected size.

EP 2 263 520 A1

FIG. 1

FIG. 2

EP 2 263 520 A1

FIG. 3

# FIG. 4

# FIG. 5

# FIG. 6

```
                    ┌─────┐
                    │ AFE │ ～ 26a
                    └─────┘
                       │
                       ▼
   ┌ ─ ─ ─ ─ ─ ─ ─ ─ ─ ─ ─ ─ ─ ─ ─ ─ ─ ─ ─ ─ ─ ─ ─ ─ ┐
   │   ╭──────────────────────────────────────╮      │
       │   MEASUREMENT LIGHT EXTRACTOR         │
   │   ╰──────────────────────────────────────╯      │
                       │        ～60
   │                   ▼                             │
       ╭──────────────────────────╮
   │   │     SIZE CALCULATOR       │                 │
       ╰──────────────────────────╯
   │                   │     ～61                     │
                       ▼
   │   ╭──────────────────────────────────────╮      │
       │  DISTANCE AND ANGLE CALCULATOR        │
   │   ╰──────────────────────────────────────╯      │
                       │      ～62                    │
   └ ─ ─ ─ ─ ─ ─ ─ ─ ─ ┼ ─ ─ ─ ─ ─ ─ ─ ─ ─ ─ ─ ─ ─ ─ ┘
            ╱          ▼
         34a       ┌─────┐
                   │ CPU │ ～30
                   └─────┘
```

# FIG. 7

# FIG. 8

```
                    ( START )
                        │
                        ▼
NO ───────────< SELECT IR MODE ? >─── S10
│                       │ YES
│                       ▼
│           ┌─────────────────────────────────┐
│           │ ACTUATE EACH SECTION AND TURN ON │── S11
│           │ IR LIGHT AND MEASUREMENT LIGHT   │
│           └─────────────────────────────────┘
│                       │
▼                       ▼
┌──────────────┐    ┌──────────────┐
│ CAPTURE IMAGE│─S12│ CAPTURE IMAGE│── S12
└──────────────┘    └──────────────┘
                        │
                        ▼
            ┌────────────────────────────┐
            │ EXTRACT MEASUREMENT LIGHT   │── S13
            └────────────────────────────┘
                        │
                        ▼
                ┌────────────────┐
                │ CALCULATE SIZE │── S14
                └────────────────┘
                        │
                        ▼
        ┌────────────────────────────┐
        │ CALCULATE DISTANCE AND ANGLE│── S15
        └────────────────────────────┘
                        │
    YES                 ▼
 ┌────< DISTANCE IS BELOW THRESHOLD VALUE ? >
 │                      │ NO            S16
 ▼                      ▼
┌────────────────┐  ┌────────────────────────┐
│ TURN OFF IR LIGHT│ │ CONTROL IR LIGHT OUTPUT │ S16
└────────────────┘  └────────────────────────┘
 S18                     │
                         │  S17
                         ▼
        ┌──────────────────────────────┐  NO
S19 ───< END ENDOSCOPY ? >──────────────────┘
                │ YES
                ▼
             ( END )
```

## FIG. 9A

## FIG. 9B

# FIG. 10

DISTANCE :
35mm

ILLUMINATION
ANGLE : 20°

Europäisches
Patentamt

European
Patent Office

Office européen
des brevets

# EUROPEAN SEARCH REPORT

**Application Number**

EP 10 16 6288

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2007/081168 A1 (JOHNSTON RICHARD S [US]) 12 April 2007 (2007-04-12) <br> * paragraph [0030] * <br> * paragraph [0035] * <br> * paragraph [0038] - paragraph [0043] * <br> * paragraph [0048] - paragraph [0049] * <br> * paragraphs [0052] - [0053] * <br> * paragraphs [0057] - [0058] * <br> * figures 6-11 * | 1,2,7,8, 10-13 | INV. <br> A61B1/07 <br> A61B5/107 <br> G01B11/02 <br> A61B1/05 <br> A61B5/00 <br><br> ADD. <br> A61B1/04 <br> A61B1/045 |
| X | EP 2 014 220 A1 (OTICON AS [DK]; WIDEX AS [DK]) 14 January 2009 (2009-01-14) <br> * paragraph [0013] * <br> * paragraph [0033] - paragraph [0034] * <br> * paragraph [0037] - paragraph [0041] * <br> * paragraphs [0060] - [0061] * <br> * figure 1 * | 1,2,7,8, 10-13 | |
| A | US 2007/123752 A1 (MELANSON JEFFREY S [US]) 31 May 2007 (2007-05-31) <br> * paragraph [0024] * | 11 | TECHNICAL FIELDS SEARCHED (IPC) |
| A | DE 36 29 435 A1 (TOSHIBA KAWASAKI KK [JP]) 12 March 1987 (1987-03-12) <br> * column 4, line 30 - column 5, line 59 * <br> * column 6, line 29 - line 46 * <br> * column 9, line 29 - column 10, line 36 * <br> * figures 1-7 * | 1,12,13 | A61B <br> G02B <br> G01B |
| A | US 4 281 931 A (CHIKAMA TOSHIO) 4 August 1981 (1981-08-04) <br> * the whole document * | 1,12,13 | |
| A | EP 0 403 399 A2 (WELCH ALLYN INC [US]) 19 December 1990 (1990-12-19) <br> * column 1, paragraph 1 * <br> * column 6, line 38 - column 8, line 11 * <br> * figures 5, 6 * | 1,12,13 | |

-/--

The present search report has been drawn up for all claims

| Place of search | Date of completion of the search | Examiner |
|---|---|---|
| Munich | 20 October 2010 | Gärtner, Andreas |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

# EUROPEAN SEARCH REPORT

Application Number

EP 10 16 6288

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 5 070 401 A (SALVATI JON R [US] ET AL) 3 December 1991 (1991-12-03) * the whole document * | 1,12,13 | |

-----

TECHNICAL FIELDS
SEARCHED      (IPC)

The present search report has been drawn up for all claims

| Place of search | Date of completion of the search | Examiner |
|---|---|---|
| Munich | 20 October 2010 | Gärtner, Andreas |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

.......................................................................................

& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 10 16 6288

This annex lists the patent family members relating to the patent documents cited in the above-mentioned European search report.
The members are as contained in the European Patent Office EDP file on
The European Patent Office is in no way liable for these particulars which are merely given for the purpose of information.

20-10-2010

| Patent document cited in search report | | Publication date | Patent family member(s) | | Publication date |
|---|---|---|---|---|---|
| US 2007081168 | A1 | 12-04-2007 | NONE | | |
| EP 2014220 | A1 | 14-01-2009 | AU | 2008274457 A1 | 15-01-2009 |
| | | | CN | 101730497 A | 09-06-2010 |
| | | | WO | 2009007178 A1 | 15-01-2009 |
| | | | US | 2010191125 A1 | 29-07-2010 |
| US 2007123752 | A1 | 31-05-2007 | NONE | | |
| DE 3629435 | A1 | 12-03-1987 | NONE | | |
| US 4281931 | A | 04-08-1981 | NONE | | |
| EP 0403399 | A2 | 19-12-1990 | DE | 69010785 D1 | 25-08-1994 |
| | | | DE | 69010785 T2 | 27-10-1994 |
| | | | JP | 2605159 B2 | 30-04-1997 |
| | | | JP | 3013805 A | 22-01-1991 |
| | | | US | 4980763 A | 25-12-1990 |
| US 5070401 | A | 03-12-1991 | NONE | | |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 8285541 A